# EUROPÄISCHE PATENTSCHRIFT

(11) **EP 1 249 251 B1**
(45) Veröffentlichungstag und Bekanntmachung des Hinweises auf die Patenterteilung: **23.06.2004**
(21) Anmeldenummer: 02007102.3
(22) Anmeldetag: 28.03.2002
(51) Int. Cl.: A61M 16/04

(54) **Beatmungstubus**
Inspiratory tube
Tube respiratoire

(30) Priorität: 12.04.2001 DE 10118605
(43) Veröffentlichungstag der Anmeldung: 16.10.2002
(73) Patentinhaber: Willy Rüsch GmbH, 71394 Kernen-Rommelshausen (DE)
(72) Erfinder: Ranzinger, Gisbert, Dr., 73773 Aichwald (DE)
(74) Vertreter: KOHLER SCHMID + PARTNER

(56) Entgegenhaltungen:
- EP-A- 0 092 618
- EP-A- 0 747 077
- AT-B- 384 738
- DE-A- 2 120 164
- GB-A- 2 168 256
- US-A- 2 862 498
- US-A- 4 090 518
- US-A- 4 231 365
- US-A- 5 873 362

## Beschreibung

Die Erfindung betrifft einen Beatmungstubus mit einer Tubuswandung zur wahlweisen Endotracheal- oder Ösophagusobturatorbeatmung mit einem ersten Lumen und einem dazu im wesentlichen parallel verlaufenden zweiten Lumen, wobei ein erster die Tubuswandung umgebender aufblasbarer Ballon im Bereich des körperzugewandten Endes des Beatmungstubus und davon beabstandet ein zweiter die Tubuswandung umgebender aufblasbarer Ballon angeordnet ist.

Ein derartiger Beatmungstubus ist beispielsweise aus der AT 384 738 bekannt geworden.

Bekannte Beatmungstuben sind häufig doppellumig aufgebaut. Eines der Lumina ist dabei an beiden Enden offen. Das andere Lumen ist an seinem körperzugewandten Ende verschlossen. Eine Beatmung über dieses Lumen wird dadurch sicher gestellt, dass in der Tubuswandung seitliche Luftöffnungen vorgesehen sind, die mit diesem Lumen in Verbindung stehen. Dieses Lumen ist ausschließlich zur Beatmung geeignet und kann keiner weiteren Verwendung zugeführt werden.

Aus der AT 384 738 wurde ein Beatmungstubus zur Ösophagus-Obturator- oder zur wahlweisen Endotracheal- oder Ösophagus-Obturator-Beatmung bekannt, der eine aufblasbare Ballonmanschette im Bereich der Tubusspitze und Luftaustrittsöffnungen in der Tubuswandung im Rachenbereich aufweist. Um den Tubus besonders in Notfallsituationen zuverlässig anwenden zu können, ist oberhalb der Luftaustrittsöffnungen ein die Tubuswandung umgebender Ballon vorgesehen, der im aufgeblasenen Zustand im wesentlichen die Form eines Torus aufweist.

Die EP 0092618 A1 offenbart einen Tubus, der ein erstes kürzeres äußeres Lumen und ein längeres, zumindest teilweise innerhalb des äußeren Lumens angeordnetes zweites Lumen aufweist. Der Tubus kann entweder in die Trachea oder in den Ösophagus eingeführt werden. Jedes Lumen weist an seiner Außenwand und nahe seines distalen Endes einen Ballon auf. Das erste Lumen ist ringförmig um das zweite, innere Lumen verlaufend ausgeführt. Der aufblasbare Ballon des ersten Lumens befindet sich im Bereich seiner distalen Mündung. Das distale Ende des Lumens ist etwas von dem Ballon beabstandet. Dieses distale Ende hält, aufgrund dessen, dass das erste Lumen um das zweite Lumen ringförmig ausgebildet ist einen Abstand zwischen der Epiglottis und der Außenwand des zweiten Lumens.

Aus der US 4,231,365 ist ein Endotrachealtubus bekannt. Bei diesem sind zwei Lumen parallel zueinander angeordnet. Beide Lumen werden von einer ersten Manschette umgeben. Das erste Lumen endet patientenseitig in unmittelbarer Nähe der ersten Manschette. Das zweite Lumen ist patientenseitig länger ausgebildet und weist an seinem patientenseitigen Ende eine zweite Manschette auf.

Auch aus der US 4,090,518 ist ein Beatmungstubus bekannt, der zwei zueinander parallel verlaufende Lumen aufweist, wobei beide Lumen von einer Manschette umgeben sind. das eine Lumen endet patientenseitig unmittelbar nach der Manschette. Das andere Lumen ist länger ausgebildet und weist in der Nähe seines patientenseitigen Endes einen Ballon auf.

### Aufgabe der Erfindung

Aufgabe der vorliegenden Erfindung ist es demgegenüber, einen Beatmungstubus zu schaffen, der vielfältiger einsetzbar ist und eine Beatmung des Patienten zuverlässig sicherstellt.

### Gegenstand der Erfindung

Erfindungsgemäß wird diese Aufgabe durch den Gegenstand des Anspruches 1 gelöst. Der erfindungsgemäße Beatmungstubus weist eine axiale Mündung des ersten Lumens unmittelbar am körperzugewandten Ende des zweiten Ballons (Oropharyngealballon) auf und der Beatmungstubus ist im Bereich des ersten Ballons einlumig ausgebildet.

Der Tubus weist dadurch ein kürzeres erstes Lumen und ein längeres zweites Lumen auf. Im Gegensatz zum Stand der Technik ist das erste Lumen an seinem körperzugewandten Ende nicht verschlossen, sondern weist dort eine axiale Mündung auf. Das erste Lumen kann somit zum einen der Beatmung und zum anderen als Führung (für medizinische Instrumente) dienen. So können beispielsweise ein Bronchoskop, ein Trachealtubus oder andere Intubationshilfsmittel durch das erste Lumen appliziert werden. Mit dem Bronchoskop kann die Inspektion der Trachea und das Absaugen von Trachealsekreten erfolgen. Zudem kann ein Führungskatheter eingeführt werden, über den bzw. entlang dem der Beatmungstubus eingeführt werden kann. Der erste Ballon dichtet wahlweise im Ösophagus oder in der Trachea ab, während der zweite Ballon immer im Rachenraum abdichtet. Die Mündung des ersten Lumens befindet sich bei eingeführtem Beatmungstubus im Bereich des Kehlkopfeingangs. Das erste Lumen kann für eine verbesserte Beatmung im Bereich zwischen dem ersten und zweiten Ballon zusätzlich seitliche Luftöffnungen in der Tubuswandung aufweisen. Für weitere Anwendungsfälle können gegebenenfalls weitere Lumina am Tubus vorgesehen sein. So kann ein weiteres Lumen in der Wandung des Beatmungstubus ausgebildet sein, das bis zur Spitze (terminales Ende) des Beatmungstubus verläuft und dort offen endet. Im körperabgewandten Abschnitt des Beatmungstubus kann dieses Lumen aus der Wandung des Beatmungstubus herausgeführt werden und in eine eigenständige Leitung (Gasabführleitung) übergehen, die mit geeigneten Prüfgeräten verbindbar ist (Kapnographie). Über diese eigenständige Leitung ist es möglich, geringe Gasvolumina aus dem Spitzenbereich des platzierten Beatmungstubus abzuziehen und über geeignete Detektionsgeräte zu analysieren (z.B. CO₂-Anteil im erfassten Gasvolumen). Über eine derartige Bestimmung der Gaszusammensetzung im erfassten Gasvolumen kann die Lage der Spitze des Beatmungstubus bestimmt werden. Es versteht sich, dass am Beatmungstubus Konnektoren zur Verbindung mit Beatmungshilfen vorgesehen sein können.

Das körperzugewandte Ende des ersten Lumens ist unmittelbar am körperzugewandten Ende des zweiten Ballons angeordnet. Durch diese Maßnahme wird zum einen sicher gestellt, dass das erste Lumen den Zugang zur Trachea nicht versperrt, sollte das zweite Lumen etwas zu weit in den Ösophagus eingeschoben werden und dass ein Intubationshilfsmittel problemlos in die Trachea eingeführt werden kann. Zum anderen stellt die Mündung des ersten Lumens und die damit verbundene Querschnittsvergrößerung des Tubus kein vergrößertes Hindernis beim Einführen des Tubus dar. Eine Reizung des Gewebes wird dadurch minimiert. Vielmehr kann der Tubus im Endabschnitt des körperzugewandten Bereichs (Spitze) mit geringerem Durchmesser ausgebildet werden, wenn der Tubus im Bereich des ersten Ballons einlumig ausgebildet ist. Dadurch wird das Einführen des Beatmungstubus erleichtert. Außerdem bedeutet dies einen höheren Komfort für den Patienten. Wenn die Mündung des ersten Lumens in unmittelbarer Nähe des zweiten Ballons angeordnet ist, können außerdem nicht aus Versehen beide Lumina in den Ösophagus eingeführt werden, was eine Beatmung verhindern könnte.

Der Abstandshalter ist als Zunge oder Steg ausgebildet und an der Außenseite des ersten Lumens befestigt. Dadurch wird ein Anlegen der Epiglottis an die Mündung und eine daraus folgende Atemwegsobstruktion vermieden.

Eine Weiterbildung zeichnet sich dadurch aus, dass der Abstandshalter als ein die Mündung überspannender Steg ausgebildet ist und zusätzlich an der Außenseite des zweiten Lumens befestigt ist. Ein oder mehrere Stege können die Epiglottis davon abhalten, die Mündung zu verschließen und dennoch mindestens eine Öffnung frei geben, durch die die Beatmung erfolgen kann. Der mindestens eine Steg ist vorzugsweise dorso-caudal angebracht, so dass der mindestens eine Steg das Einführen eines optional durch das erste Lumen eingeführten Trachealtubus in Richtung Kehlkopfeingang unterstützen kann.

Bei einer alternativen Ausführungsform ist der Abstandshalter als Zunge ausgebildet, deren freies Ende auf der Außenoberfläche des Schafts des zweiten Lumens aufliegt. Beim Einführen eines Trachealtubus oder eines anderen Intubationshilfsmittels wird das freie Ende der Zunge vom Schaft des zweiten Lumens weg gedrückt bzw. gespreizt, so dass eine Öffnung frei gegeben wird. Bei an den Schaft angelegtem Zustand lässt die Zunge jedoch genügend Freiraum zum problemlosen Strömen der Atemluft. Gleichzeitig verhindert sie, ähnlich wie der mindestens eine Steg, dass das erste Lumen durch Speisereste oder anatomische Strukturen (Weichteile) blockiert wird. Die Basis der Zunge ist an der Außenseite der Mündung des ersten Lumens angebracht bzw. angelenkt. Die Zunge verjüngt sich vorteilhafterweise zum freien Ende hin.

Bei einer Ausgestaltung der Erfindung weist der Schaft des zweiten Lumens im Mündungsbereich des ersten Lumens eine der Mündung zugewandte Erhebung auf. Diese Erhebung kann eine ungewollte Obstruktion der Mündung verhindern. Insbesondere kann sie jedoch so ausgebildet sein, dass sie ein eingeführtes Intubationshilfsmittel in Richtung Kehlkopf lenkt.

In einer bevorzugten Ausgestaltung der Erfindung verjüngt sich der Beatmungstubus jenseits des ersten Ballons bis zum freien Ende des Beatmungstubus hin. Durch diese Maßnahme weist die Tubusspitze atraumatische Eigenschaften auf. Das Einführen des Beatmungstubus kann somit durchgeführt werden, ohne dass eine Verletzung des Kehlkopfes, der Trachea oder des Ösophagus zu befürchten ist.

Vorzugsweise weist der Beatmungstubus eine Tubusspitze auf, die (füll-) federspitzenförmig ausgebildet ist.

Bei einer weiteren Ausführungsform ist der Beatmungstubus zumindest auf der körperabgewandten Seite des zweiten Ballons im Querschnitt ansatzweise oval, vorzugsweise queroval, ausgebildet. Mit einer solchen Ausbildung des Tubus kann eine die Intubation oftmals limitierende zu geringe Schneidezähnekantendistanz umgangen werden.

Ist der zweite Ballon im aufgeblasenen Zustand im Querschnitt ansatzweise queroval ausgebildet, kann eine optimale Anpassung des Ballons an die Anatomie des Patienten erfolgen, indem die Druckbelastung auf das angrenzende Gewebe des Oropharynx reduziert wird. Insbesondere ist mit einem derart gestalteten Ballon eine mittige Positionierung im Oropharynx möglich. Als Ballonmaterial ist naturlatexfreies Material, insbesondere Syntheselatex, vorgesehen.

Weitere Merkmale und Vorteile der Erfindung ergeben sich aus der nachfolgenden Beschreibung eines Ausführungsbeispiels der Erfindung, anhand den Figuren der Zeichnung, die erfindungswesentliche Einzelheiten zeigen, und aus den Ansprüchen. Die einzelnen Merkmale können je einzeln für sich oder zu mehreren in beliebiger Kombination bei einer Variante der Erfindung verwirklicht sein.

### Zeichnung

Ein Ausführungsbeispiel des erfindungsgemäßen Beatmungstubus ist in der schematischen Zeichnung dargestellt und wird in der nachfolgenden Beschreibung erläutert. Es zeigt:
- **Fig. 1**: eine Draufsicht auf einen Beatmungstubus;
- **Fig. 2**: eine Schnittdarstellung gemäß der Linie II-II der Fig. 1

### Beschreibung des Ausführungsbeispiels

Die Figuren sind sehr schematisch dargestellt, um die wesentlichen erfinderischen Merkmale zu verdeutlichen. In den Darstellungen sind die Dimensionen nur beispielhaft und nicht maßstäblich zu verstehen.

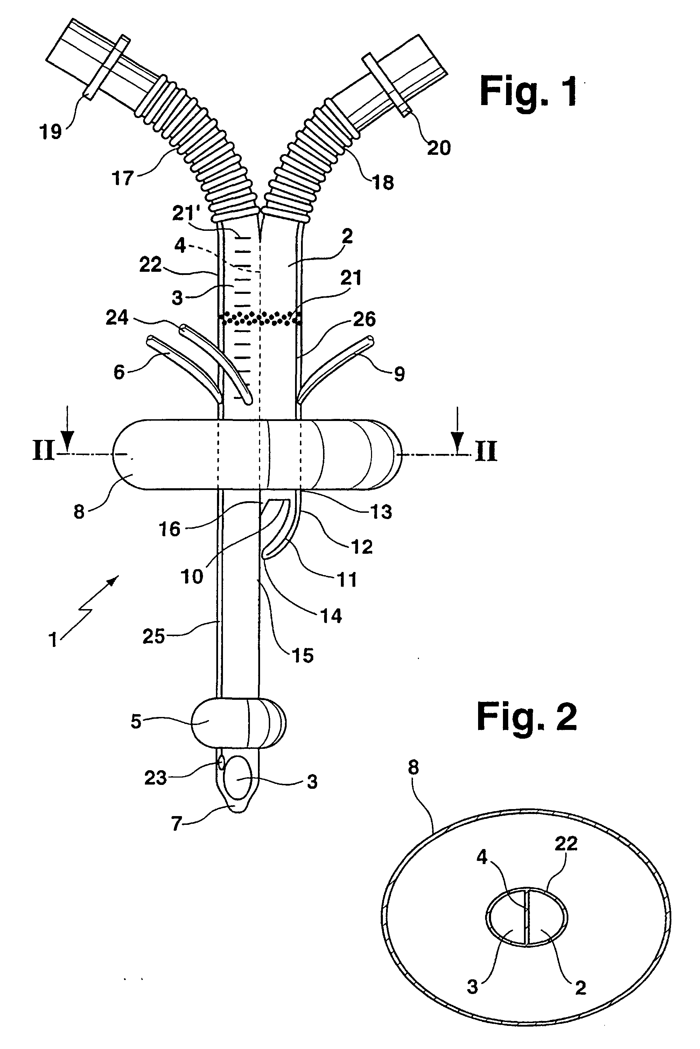

**Fig. 1** zeigt einen Beatmungstubus **1** mit einem ersten und einem zweiten Lumen **2, 3,** die im körperabgewandten Bereich des Beatmungstubus 1 durch eine Trennwand **4** voneinander getrennt sind. Im Gegensatz zum körperzugewandten Bereich ist der Beatmungstubus 1 im körperabgewandten Bereich zweilumig ausgebildet, wobei das zweite Lumen 3 am körperzugewandten Ende des Beatmungstubus 1 offen endet. Im Endbereich ist ein aufblasbarer erster Ballon **5** ausgebildet. Zum Aufblasen des ersten Ballons 5 ist eine Zuleitung **6** vorgesehen, die entlang des Beatmungstubus 1 bis zum ersten Ballon 5 geführt ist. Nach dem ersten Ballon 5 verjüngt sich die Tubusspitze **7** zum körperzugewandten offenen terminalen Ende des Beatmungstubus 1 hin und weist dadurch atraumatische Eigenschaften auf. Ferner ist das zweite Lumen 3 im körperzugewandten Bereich aus der Zeichenebene heraus leicht gekrümmt. Vom ersten Ballon 5 beabstandet ist ein zweiter aufblasbarer Ballon **8** vorgesehen, der das erste und zweite Lumen 2, 3 umgibt. Zum Aufblasen des zweiten Ballons 8 ist eine Zuleitung **9** vorgesehen. Beide Zuleitungen 6, 9 münden in eine nicht gezeigte Schnittstelle für einen Luerkonnektor. Das erste Lumen 2 endet unmittelbar am körperzugewandten Ende des zweiten Ballons 8 und weist dort eine axiale Mündung **10** auf. Im Mündungsbereich ist ein als Zunge ausgebildeter Abstandshalter **11** vorgesehen, die einenends mit ihrer Basis **12** an der Außenseite **13** der Mündung 10 elastisch befestigt ist. Das freie Ende **14** des Abstandshalters 11 ist in der Fig. 1 vom Schaft **15** des zweiten Lumens 3 gering beabstandet gezeigt, kann jedoch auch auf dem Schaft 15 aufliegen. Weiterhin ist am Schaft 15 im Mündungsbereich eine Erhebung **16** vorgesehen, die ein in das erste Lumen 2 einzuführendes Intubationshilfsmittel oder ein Bronchoskop führen kann. Am körperabgewandten Ende weisen die Lumina 2,3 Ableitungsschenkel **17, 18** auf, die vorzugsweise knickstabile Rippenschläuche sind. Um Beatmungshilfsmittel anschließen zu können, sind 15mm-Konnektoren **19, 20** vorgesehen. Eine Markierung **21** zeigt an, wie weit der Beatmungstubus 1 eingeschoben werden kann. Über eine Graduierung **21'** bzw. Skala kann die Intubationstiefe abgelesen werden. Die Abstände der Graduierungsmarkierungen betragen 1 cm. In der Wandung **22** des Beatmungstubus 1 ist ein drittes Lumen für die Kapnographie vorgesehen, das einenends in einer Öffnung **23** endet und anderenends in eine Gasabführleitung **24** übergeht, die in eine nicht gezeigte Luerschnittstelle mündet. Am Außenumfang des Beatmungstubus 1 sind röntgenstrahlendichte Markierungen **25, 26** vorgesehen, über die die Lage des platzierten Beatmungstubus 1 kontrolliert werden kann. Die Markierung 25 verläuft entlang des zweiten Lumens 3 und die Markierung 26 verläuft entlang des ersten Lumens 2. Die Markierung 26 setzt sich im beschriebenen Ausführungsbeispiel entlang des Abstandshalters 11 fort.

**Fig. 2** zeigt einen Querschnitt gemäß der Linie II-II der Fig. 1. Das erste und zweite Lumen 2, 3 sind im körperabgewandten Bereich, in dem sie im wesentlichen parallel verlaufen, durch eine Trennwand 4 voneinander getrennt. Nach Außen werden sie von der Tubuswandung 22 begrenzt. Die Querschnittsform des Beatmungstubus 1 ist in diesem Bereich vorzugsweise queroval. Die Tubuswandung 22 wird von dem zweiten Ballon 8 umgeben, der im aufgeblasenen Zustand eine ebenfalls vorzugsweise querovale Querschnittsform annimmt.

Je nach Verwendung des Beatmungstubus 1 wird das körperzugewandte Ende des Beatmungstubus 1 in die Trachea oder in den Ösophagus eingeführt. Der erste Ballon 5 wird dann aufgeblasen. Sitzt der Beatmungstubus 1 in der Trachea, erfolgt die Beatmung über das zweite Lumen 3. Sitzt der Beatmungstubus 1 dagegen im Ösophagus, dann erfolgt die Beatmung über das erste Lumen 2. Der Rachenraum wird abgedichtet, indem der zweite Ballon 8 aufgeblasen wird. Im aufgeblasenen Zustand des zweiten Ballons 8 wird der Beatmungstubus 1 in seiner platzierten Lage stabilisiert und zentriert. Über das erste Lumen 2 kann beispielsweise ein Bronchoskop in die Trachea eingeführt werden, wodurch der Abstandshalter 11 vom Schaft 15 weg gespreizt wird. Gleichzeitig kann bei Bedarf eine Magensonde über das zweite Lumen 3 eingeführt werden.

Bei einem Beatmungstubus 1 mit einer Tubuswandung 22 zur wahlweisen Endotracheal- oder Ösophagusobturatorbeatmung mit einem ersten Lumen 2 und einem dazu im wesentlichen parallel verlaufenden zweiten Lumen 3, wobei ein erster die Tubuswandung 22 umgebender aufblasbarer Ballon 5 im Bereich des körperzugewandten Endes des Beatmungstubus 1 und davon beabstandet ein zweiter die Tubuswandung 22 umgebender aufblasbarer Ballon 8 angeordnet ist, ist eine axiale Mündung 10 des ersten Lumens 2 unmittelbar am körperzugewandten Ende des zweiten Ballons 8 angeordnet und ist der Beatmungstubus 1 im Bereich des ersten Ballons 5 einlumig ausgebildet. Das Einführen von Intubationshilfsmitteln über das erste Lumen 2 wird dadurch ermöglicht, so dass der Beatmungstubus vielfältig einsetzbar ist.

## Patentansprüche

1. Beatmungstubus (1) mit einer Tubuswandung (22) zur wahlweisen Endotracheal- oder Ösophagusobturatorbeatmung mit einem ersten Lumen (2) und einem dazu im wesentlichen parallel verlaufenden zweiten Lumen (3), wobei ein erster die Tubuswandung (22) umgebender aufblasbarer Ballon (5) im Bereich des körperzugewandten Endes des Beatmungstubus (1) und davon beabstandet ein zweiter die Tubuswandung (22) umgebender aufblasbarer Ballon (8) angeordnet ist, eine axiale Mündung (10) des ersten Lumens (2) unmittelbar am körperzugewandten Ende des zweiten Ballons (8) angeordnet ist und der Beatmungstubus (1) im Bereich des ersten Ballons (5) einlumig ausgebildet 1, **dadurch gekennzeichnet, dass** im Bereich der Mündung (10) des ersten Lumens (2) mindestens ein Abstandshalter (11) zur Vermeidung einer Atemwegsobstruktion vorgesehen ist, dergestalt, dass der Abstandshalter (11) als Zunge oder Steg ausgebildet ist, die oder der an der Außenseite der Mündung des ersten Lumens (2) befestigt ist und ein Anlegen der Epiglottis an die Mündung des ersten Lumens (2) vermeidet.

2. Beatmungstubus nach Anspruch 1, **dadurch gekennzeichnet, dass** der Abstandshalter (11) als ein die Mündung (10) überspannender Steg ausgebildet ist und zusätzlich am Schaft (15) des zweiten Lumens (3) befestigt ist.

3. Beatmungstubus nach Anspruch 1, **dadurch gekennzeichnet, dass** der Abstandshalter (11) als zunge ausgebildet ist, deren freies Ende (14) auf der Außenoberfläche des Schafts (15) des zweiten Lumens (3) aufliegt.

4. Beatmungstubus nach einem der vorhergehenden Ansprüche, **dadurch gekennzeichnet, dass** der Schaft (15) des zweiten Lumens (3) im Mündungsbereich des ersten Lumens (2) eine der Mündung (10) zugewandte Erhebung (16) aufweist.

5. Beatmungstubus nach einem der vorhergehenden Ansprüche, **dadurch gekennzeichnet, dass** der Beatmungstubus (1) sich jenseits des ersten Ballons (5) bis zum freien Ende des Beatmungstubus (1) hin verjüngt.

6. Beatmungstubus nach einem der vorhergehenden Ansprüche, **dadurch gekennzeichnet, dass** der Beatmungstubus (1) eine Tubusspitze (7) aufweist, die federspitzenförmig ausgebildet ist.

7. Beatmungstubus nach einem der vorhergehenden Ansprüche, **dadurch gekennzeichnet, dass** der Beatmungstubus (1) zumindest auf der körperabgewandten Seite des zweiten Ballons (8) im Querschnitt ansatzweise oval, insbesondere queroval, ausgebildet ist.

8. Beatmungstubus nach einem der vorhergehenden Ansprüche, **dadurch gekennzeichnet, dass** der zweite Ballon (8) im aufgeblasenen Zustand im Querschnitt ansatzweise queroval ausgebildet ist.

## Claims

1. Resuscitation tube (1) comprising a tube wall (22) for alternative artificial endotracheal or esophageal obturator respiration, with a first lumen (2) and a second lumen (3) extending substantially parallel thereto, wherein a first inflatable balloon (5) surrounding the tube wall (22) is disposed in the region of the end of the resuscitation tube (1) facing the body, and a second inflatable balloon (8) surrounding the tube wall (22) is disposed at a separation from the first inflatable balloon (5), an axial opening (10) of the first lumen (2) is disposed directly at the end of the second balloon (8) facing the body and the resuscitation tube (1) is formed with one lumen in the region of the first balloon (5), **characterized in that** at least one spacer (11) is provided in the region of the opening (10) of the first lumen (2) to prevent obstruction of the respiratory tract, such that the spacer (11) is designed as a tongue or a bar mounted to the outer side of the opening of the first lumen (2) and preventing abutment of the epiglottis to the opening of the first lumen (2).

2. Resuscitation tube according to claim 1, **characterized in that** the spacer (11) is formed as a bar overlapping the opening (10) and is additionally mounted to the shaft (15) of the second lumen (3).

3. Resuscitation tube according to claim 1, **characterized in that** the spacer (11) is formed as a tongue whose free end (14) is supported on the outer surface of the shaft (15) of the second lumen (3).

4. Resuscitation tube according to any one of the preceding claims, **characterized in that** the shaft (15) of the second lumen (3) has an elevation (16) in the opening region of the first lumen (2), which faces the opening (10).

5. Resuscitation tube according to any one of the preceding claims, **characterized in that** the resuscitation tube (1) tapers beyond the first balloon (5) to the free end of the resuscitation tube (1).

6. Resuscitation tube according to any one of the preceding claims, **characterized in that** the resuscitation tube (1) has a tube tip (7) which is formed like the tip of a fountain pen.

7. Resuscitation tube according to any one of the preceding claims, **characterized in that** the resuscitation tube (1) has a basically oval, in particular transversely oval cross-section, at least on the side of the second balloon (8) facing away from the body.

8. Resuscitation tube according to any one of the preceding claims, **characterized in that** the second balloon (8) has a basically transversely oval cross-section when inflated.

## Revendications

1. Tube respiratoire (1) présentant une paroi de tube (22) et destiné à réaliser sélectivement une respiration ou ventilation assistée par voie endo-trachéale ou avec obturation de l'oesophage, comportant une première lumière (2) et une seconde lumière (3) s'étendant sensiblement de manière parallèle à la première, un premier ballonnet gonflable (5) qui entoure la paroi de tube (22) étant disposé dans la zone de l'extrémité du tube respiratoire (1), dirigée vers le corps du patient, et un second ballonnet gonflable (8) qui entoure la paroi de tube (22) étant disposé à distance du premier, un orifice de sortie axial (10) de la première lumière (2) étant disposé à l'extrémité du second ballonnet (8), dirigée vers le corps du patient, et le tube respiratoire (1) étant d'une configuration à une seule lumière dans la zone du premier ballonnet (5),
**caractérisé en ce que** dans la zone de l'orifice de sortie (10) de la première lumière (2) il est prévu au moins un écarteur (11) pour empêcher une obstruction de voie respiratoire, de façon telle que l'écarteur (11) soit réalisé en tant que languette ou nervure, qui est fixée sur le côté extérieur de l'orifice de sortie de la première lumière (2) et empêche l'épiglotte de venir s'appliquer sur l'orifice de sortie de la première lumière (2).

2. Tube respiratoire selon la revendication 1, **caractérisé en ce que** l'écarteur (11) est réalisé en tant que nervure surmontant l'orifice de sortie (10), et est en plus fixé sur le corps tubulaire (15) de la seconde lumière (3).

3. Tube respiratoire selon la revendication 1, **caractérisé en ce que** l'écarteur (11) est réalisé en tant que languette dont l'extrémité libre (14) s'appuie sur la surface extérieure du corps tubulaire (15) de la seconde lumière (3).

4. Tube respiratoire selon l'une des revendications précédentes, **caractérisé en ce que** le corps tubulaire (15) de la seconde lumière (3) présente, dans la zone d'embouchure ou de l'orifice de sortie de la première lumière (2), une protubérance (16) dirigée vers l'orifice de sortie (10).

5. Tube respiratoire selon l'une des revendications précédentes, **caractérisé en ce que** le tube respiratoire (1) se rétrécit au-delà du premier ballonnet (5) jusqu'à l'extrémité libre du tube respiratoire (1).

6. Tube respiratoire selon l'une des revendications précédentes, **caractérisé en ce que** le tube respiratoire (1) présente une pointe de tube (7) qui est réalisée en forme de pointe de stylo à plume.

7. Tube respiratoire selon l'une des revendications précédentes, **caractérisé en ce que** le tube respiratoire (1), au moins sur le côté du second ballonnet (8), qui s'éloigne du corps du patient, est réalisé en section transversale, avec une forme originellement ovale, notamment ovale transversale.

8. Tube respiratoire selon l'une des revendications précédentes, **caractérisé en ce que** le second ballonnet (8), dans l'état gonflé, est réalisé, en section transversale, avec une forme originellement ovale transversale.
